# EUROPÄISCHE PATENTSCHRIFT

(11) **EP 1 207 878 B1**
(45) Veröffentlichungstag und Bekanntmachung des Hinweises auf die Patenterteilung: **19.02.2003**
(21) Anmeldenummer: 00960527.0
(22) Anmeldetag: 26.08.2000
(51) Int. Cl.: A61K 31/385, A61P 25/06

(54) **BEHANDLUNG DER MIGRÄNE DURCH VERABREICHUNG VON ALPHA-LIPONSÄURE ODER DERIVATEN DERSELBEN**
TREATMENT OF MIGRAINE BY THE ADMINISTRATION OF ALPHA-LIPOIC ACID OR DERIVATIVES THEREOF
TRAITEMENT DE LA MIGRAINE PAR ADMINISTRATION D'ACIDE ALPHA-LIPOIQUE OU DE DERIVES DE CET ACIDE

(30) Priorität: 30.08.1999 DE 19941217
(43) Veröffentlichungstag der Anmeldung: 29.05.2002
(73) Patentinhaber: VIATRIS GmbH & Co. KG, 60314 Frankfurt am Main (DE)
(72) Erfinder: SCHOENEN, Jean, B-4052 Beaufays-Chaudfontaine (BE); ENGEL, Jürgen, 63755 Alzenau (DE); WESSEL, Klaus, 61118 Bad Vilbel (DE); PEUKERT, Manfred, 59581 Warstein (DE); LOBISCH, Michael, 61203 Reichelsheim/Blofeld (DE); BORBE, Harald, 55127 Mainz (DE)
(74) Vertreter: Wibbelmann, Jobst, Dr., Dipl.-Chem.
(86) Internationale Anmeldenummer: EP0008315
(87) Internationale Veröffentlichungsnummer: WO01015693

(56) Entgegenhaltungen:
- WO-A-94/00135
- WO-A-99/51097
- DE-A- 4 327 462
- US-A- 5 569 670
- LOW: "symptomatic treatment..." J. OF THE AM. MED. ASSOCIATION, Bd. 280, Nr. 21, Seiten 1863-1864, XP000997203
- VARIOUS: "the merck manual" 1999 , MERCK RESEARCH LABORATORIES , N.J. XP002165174 Seite 1376, Spalte 2, Absatz 3 - Absatz 5
- GLEITER C H ET AL: "LACK OF INTERACTION BETWEEN THIOCTIC ACID, GLIBENCLAMIDE AND ACARBOSE" BRITISH JOURNAL OF CLINICAL PHARMACOLOGY,GB,BLACKWELL SCIENTIFIC PUBL, Bd. 48, Nr. 6, 1999, Seiten 819-825, XP000908779 ISSN: 0306-5251
- DATABASE WPI Section Ch, Week 197924 Derwent Publications Ltd., London, GB; Class B05, AN 1979-45483B XP002165175 & SU 620 265 A (PHYSIOTHERAPY RES), 7. Juli 1978 (1978-07-07)

## Beschreibung

Die Erfindung betrifft die Verwendung von racemischer α-Liponsäure oder deren Enantiomeren oder pharmazeutisch akzeptablen Salzen, Amiden, Estern oder Thioestern derselben, in reduzierter oder oxidierter Form, zur Herstellung eines Arzneimittels zur Vorbeugung oder akuten oder chronischen Behandlung von Migräne.

Die Migräne ist eine der am häufigsten vorkommenden Erkrankungen. Migräne wird von der International Headache Society (IHS) als eine Erkrankung definiert, die durch anfallsweise Attacken von Kopfschmerz kombiniert mit vegetativen Beschwerden charakterisiert ist. Die schmerzhaften Episoden treten akut und episodisch auf, aber die Erkrankung selbst muss als chronisch angesehen werden. Bei einigen Patienten ohne die sogenannte Aura dauern die Schmerzattacken etwa 4 bis 72 Stunden, sind oft halbseitig und verbunden mit Übelkeit und Erbrechen sowie Photo- und Phonophobie. Bei Patienten mit der sogenannten Aura treten einige Minuten vor einer Schmerzattacke reversibel neurologische Anzeichen wie Aphasie, Parese, Ataxie und Schwindel auf. Nach epidemiologischen Studien leiden etwa 8 bis 15 % der Bevölkerung gelegentlich oder häufig an milden bis schweren Migräneanfällen. Frauen sind gewöhnlich mehr betroffen als Männer. Migräne tritt üblicherweise im Alter von 20 bis 35 Jahren das erste Mal auf und ist bei Kindern seltener als bei Erwachsenen. Die Diagnose wird vom Arzt nur mittels Anamnese und klinischen Daten vorgenommen. Technische und biochemische Verfahren, die eine zuverlässige Diagnose liefern, gibt es nicht.

Die Pathophysiologie und Pathobiochemie der Migräne ist unterentwickelt. In früheren Zeiten wurde die Migräne als psychosomatische Erkrankung ohne biologischen Hintergrund angesehen und es war unklar, ob Migräne eine körperliche Erkrankung oder eine psychische Gesundheitsstörung ist. Aber heute wird dies von den meisten Experten nicht mehr als zweifelhaft angesehen. Jedoch muss immer noch berücksichtigt werden, dass psychische Veränderungen die Migräne auslösen können und viele andere divergierende Faktoren wie hormonale (Menstruation), nutritive (Alkohol und Fehlernährung), medizinische (Arzneimittel), umweltbedingte (Lärm) und psychische (Stress) zur Migräne beitragen. Immer noch suchen Patienten halbprofessionelle Hilfe wie Homöopathie oder Zelltherapie.

Bei einigen Patienten war eine Verminderung des cerebralen Blutflusses in isolierten Regionen des Gehirns mit Migräneanfällen verbunden (Lauritzen & Hansen, 1988). Perivasculäre Veränderungen, die afferente schmerzleitende Nervenfasern stimulieren, wurden zumindest bei einigen Patienten als zur Migräne beitragend angesehen (Moskowitz A.M. et al., Rev Neurol 145: 181-195; 1989), ebenso Veränderungen von verschiedenen Neurotransmittern (Noradrenalin, Serotonin, Tachykinine etc.) (Edvinsson L. et al., in: Olesen J. Edvinsson L (ed.) Basic mechanisms of headache. Elsevier Science Publishers, Amsterdam 129-144; 1988). Eine Verminderung des mitochondrialen Phosphorylierungspotentials im Gehirn von Migränepatienten wurde vor kurzem diskutiert, jedoch bleibt offen, ob diese Beobachtung primäre Defizite aufzeigt oder nur ein sekundäres Vorkommnis in der Pathophysiologie der Migräne darstellt (Schoenen et al., Neurology 50: 466-470; 1998).

Bis heute wird Migräne nur symptomatisch behandelt. Einige Medikationen werden dazu verwendet, sogenannte Triggerfaktoren der Migräneanfällen zu modulieren (β-Blocker). Andere Verbindungen wirken vasoaktiv (Serotoninantagonisten wie Sumatriptan, nichtsteroidale Antirheumatika wie Acetylsalicylicsäure, Anticonvulsiva wie Valproinsäure). Die vasoaktive Wirkung von Ergotamin trägt jedoch nicht zu seinen klinischen Effekten bei. Vitamine B₂ ist ein wichtiger Kofaktor in der Atmungskette der Mitochondrien und reduziert Berichten zufolge das Auftreten von Migräneanfällen in 68 % der Patienten (Schoenen J. et al., aao.). Es wurden jedoch weder ein Vitamin B₂-Mangel bei Migränepatienten beobachtet noch zeigen Patienten mit einem Vitamin B₂-Mangel ein klinisches Bild, das der Migräne vergleichbar ist.

Alle diese möglichen Interventionen sind pragmatische Ansätze, zumindest bei einigen Patienten die Beschwerden von akuten Migräneanfällen zu lindern. Verschiedene Kombinationen von Arzneimitteln und anderen Behandlungen werden individuell verwendet, um einen klinischen Nutzen zu erzielen. Arzneimittel werden entweder akut zur Linderung eines Migräneanfalls oder chronisch zur Verminderung der Häufigkeit der Anfälle angewendet. Die Eliminierung von medikamentösen oder umweltbedingten Triggerfactoren ist ebenfalls ein wichtiger Versuch, den Patienten zu helfen. Es gibt jedoch noch keine Behandlung der zugrundeliegenden Erkrankung Migräne selbst, was durch das fehlende Verständnis der Pathophysiologie der Migräne erklärt werden kann. Der Nutzen pragmatischer Therapien ist für viele leidende Patienten wertvoll, diese können aber bis heute nicht von ihrer Erkrankung geheilt werden.

α-Liponsäure ist ein in der Natur vorkommendes Antioxidans und ein Kofaktor der Glucose metabolisierenden Pyruvatdehydrogenase (Packer L. et al., Free Radicals in Biology & Medicine 19 (2): 227-250; 1995) und wird in breitem Umfang für die Behandlung der diabetischen Polyneuropathie (Ziegler D. et al., Diabetologia 38: 1425-1433; 1995) verwendet. Darüber hinaus wurde α-Liponsäure jahrzehntelang für die Behandlung von Lebererkrankungen (Bode J.Ch. et al., DMW 112 (9), 349-352; 1987) und Pilzvergiftungen (Brunn J. et al., Internist. Prax. 19: 475-478, 1979) verwendet. Die molekulare Wirkungsweise wurde vor kurzem als die eines diabetesspezifischen Antioxidans charakterisiert (Nagamatsu M. et al., Diabetes Care 18 (8): 1160-1167; 1995).

Die biologischen und therapeutischen Effekte der α-Liponsäure in oxidierter und reduzierter Form finden sich auch bei zahlreichen Derivaten und Metaboliten in teils abgeschwächter, teils verbesserter Form (zum Beispiel bei 3-Ketoliponsäure, 1,2-Diselenolan-3-Pentansäure, Liponamid, Octotiamin, 2-(NNdimethylamine)ethylamidolipoate-HCI, Tocopheryl- und tocotrienyl-Lipoat, gamma-hydroxybutyrat-Lipoat, Liponsäure-Vitamin E-Ester, N-Acetyl-p-Aminophenol-Derivate der Liponsäure und andere) (Tirosh O. Sen CK, Roy S, Kobayashi S, Packer L. Neuroprotective effects of α-lipoic acid and ist positively charged amide analogue. Free Rad Biol Med 26 (11/12), 1418-1426, 1999); EP 0 855 396 A1, EP 0 869 126 A1, PCT/GB98/02155, WO 99/06040, DE 43 27 462 A1).
Diese Derivate wurden vorgeschlagen, um den Metabolismus und die Verteilung in vivo zu verbessern, was auch für die Verteilung in das zentrale Nervensystem gelten kann. Einzelne Derivate können auch die Effekte (z.B. Affinität und die Umsatzrate) an den biologischen Targets (biologische Redoxsysteme wie α-Ketosäuren-Dehydrogenasen, H-Protein, Thioredoxin, Glutathionreduktase oder zelluläre Redoxsysteme wie Glutathion, Ubichinon, Komplex I der Atmungskette, oder redox- und SH-sensitive Proteine und Enzyme, das NO-System, Katalase, der zelluläre Cystin/Cystein-Shuttle, Homocystein, Tyrosinkinase, MAP-Kinase, Metallionen (zur Komplexbildung), alpha 1-Antiproteinase, oder redoxsensitive Transkriptionsfaktoren wie NF-kB oder AP1) von α-Liponsäure verbessern oder andere aktive Moleküle mit dem Ziel synergistischer oder additiver pharmakologischer Wirkung an α-Liponsäure koppeln.

Deshalb wird der Ausdruck "α-Liponsäure" in dieser Schrift als ein genereller Ausdruck verwendet, der außer den Enantiomeren, dem Racemat und Gemischen der Enantiomeren auch Derivate (Ester, Thioester, Ether, Salze, Amide, Metaboliten etc) abdeckt, soweit die aktive Dithiolangruppe der α-Liponsäure für die biologische und medizinische Wirkung des Derivates weiterhin mit verantwortlich ist.

Arzneimittel mit α-Liponsäure sind seit Jahrzehnten erhältlich und gut verträglich. In dieser Zeit wurden viele Anwendungsmöglichkeiten getestet, aber es wurde nie von einem Nutzen bei der Behandlung der Migräne berichtet.

Ziel der Erfindung ist die Verbesserung des Gesundheitszustands von Migränepatienten.

Diese Aufgabe wird durch Verwendung von racemischer α-Liponsäure oder deren Enantiomeren oder pharmazeutisch akzeptablen Salzen, Amiden, Estern oder Thioestern derselben, in reduzierter oder oxidierter Form, als Wirkstoff bei der Vorbeugung oder der akuten oder chronischen Behandlung von Migräne gelöst.

Der Nutzen liegt in einer Reduktion der Schwere und, noch wichtiger, der Häufigkeit von Migräneanfällen. Im günstigsten Fall wird durch die chronische Verwendung von α-Liponsäure oder deren obengenannter Derivate eine vollständige Heilung der Migräne durch Verschwinden jeglicher Anfälle möglich.

Ein weiterer wichtiger Vorteil der Erfindung liegt in der sehr guten Verträglichkeit der verwendeten Wirkstoffe.

Der Wirkstoff kann in einem oral oder parenteral zu verabreichenden Arzneimittel formuliert oder in der Art eines Nahrungsergänzungsmittels oder medizinischen Nahrungsmittels für die parenterale Ernährung verabreicht werden.

Geeignete Zubereitungen sind aus der Patentliteratur bekannt und z.B. in folgenden Schriften beschrieben:
EP 0858 802 A2
EP 0318 891 A1
EP 0 560 092 B1
US 5,650,429 A
US 5,334,612 A
US 5,569,670 A

Die so hergestellten Produkte können für den Zweck des Einsatzes beschriftet in den Verkehr gebracht werden, wobei für die Anleitung der Anwendung für Fachpersonal und Patient die entsprechenden nationalen Regeln zu beachten sind. Die Produkte können sich hierbei üblicherweise im Rechtsrahmen von Arzneimitteln oder gegebenenfalls auch von Nahrungsergänzungsstoffen bewegen.

Die Dosierung des Wirkstoffs liegt üblicherweise im Bereich von 100 bis 1800, vorzugsweise 200 bis 1200, insbesondere 200 bis 600, mg racemischer α-Liponsäure pro Tag oder, bezogen auf den Dithiolanrest als Basis, einer äquivalenten Dosis eines der anderen Wirkstoffe, wobei diese Gesamtdosis einmal täglich oder verteilt auf zwei oder drei Tagesdosen verabreicht wird.

Derivate der α-Liponsäure in reduzierter oder oxidierter Form (z.B. Salze, Ester, Thioester, Ether, Amide, Metabolite) können analog eingesetzt werden, soweit sie in einer Dosis verabreicht werden, dass äquivalente Konzentrationen oder biologische Effekte an den Zielstrukturen (biologische Redoxsysteme) erreicht werden.

Vorzugsweise wird optisch rechtsdrehende α-Liponsäure (R(+)-α-Liponsäure oder R(-)-Dihydroliponsäure) eingesetzt oder Derivate.

Eine weitere bevorzugte Ausführungsform der Erfindung besteht in der Verabreichung des Wirkstoffs in freier oder fixer Kombination mit einer weiteren für die Behandlung von Migräne verwendeten Substanz oder mehreren.

Bevorzugte Beispiele für Kombinationspartner sind Sumatriptan oder eine andere Verbindung der Triptangruppe, Ergotamin oder ein Derivat desselben, ein β-Blocker, ein Anticonvulsivum, ein Anatgetikum, ein Antiemetikum oder ein Calciumantagonist ist, ohne dass die Erfindung auf diese beschränkt ist. Ebenso vorteilhaft ist die Verabreichung des Wirkstoff als freie oder fixe Kombination mit Vitaminen, Antioxidantien und/oder biologisch funktionellen Kofaktoren. Besonders bevorzugt ist dabei Vitamin B₂.

Die Erfindung wird im folgenden anhand von Beispielen erörtert, ohne auf diese beschränkt zu sein.

### Pharmazeutische Beispiele

### Beispiel 1:

### Tabletten mit 600 mg racemischer α-Liponsäure

1200 g racemische α-Liponsäure mit Partikelgröße-60 % > 100 µm werden mit 120 g Hydroxypropylcellulose, niedrig substituiert (L-HPC-LH 22/Shin Etsu) gemischt und die Mischung mit 600 g gereinigtem Wasser befeuchtet und durchgearbeitet.

Nach Passage durch ein Sieb der Maschenweite 2 mm wird das Granulat getrocknet, nochmals durch ein Sieb der Maschenweite 1 mm gesiebt und nach Zumischen von 48 g Magnesiumstearat zu Tabletten in Oblong-Format vom Gewicht 684 mg, einer Länge von 18 mm, einer Breite von 8 mm und einem Wölbungsradius von 6 mm gepresst. Eine Tablette enthält 600 mg racemische α-Liponsäure.

Die Tabletten können anschließend nach üblichen Standardverfahren mit einem magensaftlöslichen oder magensaftpermeablen Filmüberzug versehen werden.

### Beispiel 2:

### Ampullen mit 200 mg racemischer α-Liponsäure als Trometamolsalz in 10 ml

250 g racemische α-Liponsäure werden zusammen mit 352,3 g Trometamol (2-Amino-2-(hydroxymethyl)-1,3-propandiol) in einer Mischung aus 9 Liter Wasser für Injektionszwecke und 200 g 1,2-Propylenglykol unter Rühren gelöst. Die Lösung wird mit Wasser für Injektionszwecke auf 12,5 Liter aufgefüllt und anschließend durch ein Membranfilter der Porenweite 0,2 µm mit Glasfaservorfilter filtriert. Das Filtrat wird unter aseptischen Bedingungen zu 10 ml in sterilisierte 10 ml Ampullen abgefüllt. Eine Ampulle enthält in 10 ml Injektionslösung 200 mg racemische α-Liponsäure als Trometamolsalz.

### Klinische Beispiele

Racemische α-Liponsäure wurde an Migränepatienten oral in einer Tagesdosis von 200-600 mg akut und chronisch in Form handelsüblicher Darreichungsformen verschiedener Stärken verabreicht. Der Wirkstoff wurde entweder als Einmalgabe am Morgen oder nach Bedarf während des Tages
verabreicht. Die Häufigkeit und Schwere von Migräneanfällen wurde mit dem Zeitraum vor der Behandlung verglichen. Der Wirkstoff wurde entweder unbehandelten oder bereits in Therapie befindlichen Patienten, die vorher mit anderen Wirkstoffen gegen Migräne behandelt wurden und auf ihre bisherige Behandlung nicht gut ansprachen, verabreicht.

| Patient Behandlung - | | Klinischer Effekt |
|---|---|---|
| 1 | Keine Vorbehandlung | 50 % Reduktion der Häufigkeit von Migräneattacken |
| 2 | Vorbehandlung mit Valproat, danach ersetzt durchα-Liponsäure | Effekt der Behandlung mit Valproat bleibt nach dem Ersetzen erhalten |
| 3 | Vorbehandlung mit Vitamin B₂ | 100 % Reduktion der Häufigkeit von Migräneattacken |
| 4 | Vorbehandlung mit Vitamin B₂ | 100 % Reduktion der Häufigkeit von Migräneattacken |
| 5 | Vorbehandlung mit Valproat | 50 % Reduktion der Häufigkeit von Migräneattacken |
| 6 | Vorbehandlung mit Valproat, Vitamin B₂ und Acetylsalicylsäure | 80 % Reduktion der Häufigkeit von Migräneattacken |
| 7 | Vorbehandlung mit Valproat | 50 % Reduktion der Häufigkeit von Migräneattacken |
| 8 | Vorbehandlung mit Valproat | 50 % Reduktion der Schwere von Migräneattacken |
| 9 | Keine Vorbehandlung | 75 % Reduktion der Häufigkeit von Migräneattacken |
| 10 | Vorbehandlung mit Cyclandelat | 40 % Reduktion der Häufigkeit von Migräneattacken |
| 11 | Keine Vorbehandlung | 30-100 % Reduktion der Schwere von Attacken am Tag nach der Einnahme von 200-600 mg α-Liponsäure |

Zur Beurteilung dieser Beobachtungen bei der medizinischen Behandlung von Migränepatienten muss in Betracht gezogen werden, dass das Ausmaß der gefundenen Effekte, gemessen an den Erfahrungen mit anderen Therapien, sehr bemerkenswert ist. Insbesondere die Wirkung bei anderenfalls therapieresistenten Patienten muss als beachtlich und überraschend bezeichnet werden. Dies unterstreicht den Wert der Erfindung für die zukünftige Behandlung der Migräne.

## Patentansprüche

1. Verwendung von racemischer α-Liponsäure oder deren Enantiomeren oder pharmazeutisch akzeptablen Salzen, Amiden, Estern oder Thioestern derselben in reduzierter oder oxidierter Form, zur Herstellung eines Medikamentes zur Vorbeugung oder akuten oder chronischen Behandlung von Migräne.

2. Verwendung nach Anspruch 1, wobei die Schwere oder Häufigkeit von Migräneattacken vermindert wird.

3. Verwendung nach Anspruch 1 oder 2, wobei der Wirkstoff in Form eines Arzneimittels vorliegt.

4. Verwendung nach Anspruch 1 oder 2, wobei der Wirkstoff in Form eines Nahrungsmittels oder Nahrungsergänzungsmittels für die parenterale Ernährung vorliegt.

5. Verwendung nach Anspruch 1 oder 2, wobei der Wirkstoff in einer Dosierung von 100 bis 1800 mg racemischer α-Liponsäure pro Tag oder, bezogen auf den Dithiolanrest als Basis, einer äquivalenten Dosis eines der anderen Wirkstoffe verwendet wird.

6. Verwendung nach Anspruch 1 oder 2, wobei der Wirkstoff in einer Dosierung von 200 bis 1200 mg racemischer α-Liponsäure pro Tag oder, bezogen auf den Dithiolanrest als Basis, einer äquivalenten Dosis eines der anderen Wirkstoffe verwendet wird.

7. Verwendung nach Anspruch 1 oder 2, wobei der Wirkstoff in einer Dosierung von 200 bis 600 mg racemischer α-Liponsäure pro Tag oder, bezogen auf den Dithiolanrest als Basis, einer äquivalenten Dosis eines der anderen Wirkstoffe verwendet wird.

8. Verwendung nach einem der Ansprüche 5 bis 7, wobei die Dosierung einmal täglich oder verteilt auf zwei oder drei Tagesdosen verabreicht wird.

9. Verwendung nach Anspruch 1, wobei der Wirkstoff als freie oder fixe Kombination mit einer weiteren für die Behandlung von Migräne verwendeten Substanz oder mehreren verabreicht wird.

10. Verwendung nach Anspruch 9, wobei die weitere Substanz Sumatriptan oder eine andere Verbindung der Triptangruppe, Ergotamin oder ein Derivat desselben, ein β-Blocker, ein Anticonvulsivum, ein Analgetikum, ein Antiemetikum oder ein Calciumantagonist ist.

11. Verwendung nach Anspruch 1, wobei der Wirkstoff als freie oder fixe Kombination mit Vitaminen, Antioxidantien und/oder biologisch funktionellen Kofaktoren verabreicht wird.

## Claims

1. Use of racemic α-lipoic acid or its enantiomers or pharmaceutically acceptable salts, amides, esters or thioesters thereof, in reduced or oxidized form, for the manufacture of a medicament for prevention or acute or chronic treatment of migraine.

2. Use according to Claim 1, where the severity or frequency of migraine attacks is reduced.

3. Use according to Claim 1 or 2, where the active ingredient is in the form of a pharmaceutical.

4. Use according to Claim 1 or 2, where the active ingredient is in the form of a food or food supplement for parenteral nutrition.

5. Use according to Claim 1 or 2, where the active ingredient is used in a dosage of 100 to 1800 mg of racemic α-lipoic acid per day or, based on the dithiolane residue, an equivalent dose of one of the other active ingredients.

6. Use according to Claim 1 or 2, where the active ingredient is used in a dosage of 200 to 1200 mg of racemic α-lipoic acid per day or, based on the dithiolane residue, an equivalent dose of one of the other active ingredients.

7. Use according to Claim 1 or 2, where the active ingredient is used in a dosage of 200 to 600 mg of racemic α-lipoic acid per day or, based on the dithiolane residue, an equivalent dose of one of the other active ingredients.

8. Use according to any of Claims 5 to 7, where the dosage is administered once a day or divided into 2 or 3 daily doses.

9. Use according to Claim 1, where the active ingredient is administered as free or fixed combination with another substance, or several, used for treating migraine.

10. Use according to Claim 9, where the other substance is sumatriptan or another compound from the triptan group, ergotamine or a derivative thereof, a β blocker, an anticonvulsant, an analgesic, an antiemetic or a calcium channel blocker.

11. Use according to Claim 1, where the active ingredient is administered as free or fixed combination with vitamins, antioxidants and/or biologically functional cofactors.

## Revendications

1. Utilisation d'acide α-lipoïque racémique ou de ses énantiomères ou bien des sels, amides, esters ou thioesters pharmaceutiquement acceptables de ceux-ci, sous forme réduite ou oxydée, pour préparer un médicament destiné à la prévention ou bien au traitement aigu ou chronique de la migraine.

2. Utilisation selon la revendication 1, dans laquelle la gravité ou la fréquence des accès de migraine est réduite.

3. Utilisation selon la revendication 1 ou la revendication 2, dans laquelle le principe actif est présent sous la forme d'un médicament.

4. Utilisation selon la revendication 1 ou la revendication 2, dans laquelle le principe actif est présent sous la forme d'un aliment ou d'un complément nutritif pour l'alimentation parentérale.

5. Utilisation selon la revendication 1 ou la revendication 2, dans laquelle le principe actif est utilisé selon un dosage de 100 à 1800 mg par jour d'acide α-lipoïque racémique ou bien, ramené au dithiolane résiduel comme base, d'une dose équivalente de l'un des autres principes actifs.

6. Utilisation selon la revendication 1 ou la revendication 2, dans laquelle le principe actif est utilisé selon un dosage de 200 à 1200 mg par jour d'acide α-lipoïque racémique ou bien, ramené au dithiolane résiduel comme base, d'une dose équivalente de l'un des autres principes actifs.

7. Utilisation selon la revendication 1 ou la revendication 2, dans laquelle le principe actif est utilisé selon un dosage de 200 à 600 mg par jour d'acide α-lipoïque racémique ou bien, ramené au dichiolane résiduel comme base, d'une dose équivalente de l'un des autres principes actifs.

8. Utilisation selon l'une des revendications 5 à 7, dans laquelle le dosage est administré une fois par jour ou bien réparti en deux ou trois doses journalières.

9. Utilisation selon la revendication 1, dans laquelle le principe actif est administré sous la forme d'une combinaison libre ou fixe avec une ou plusieurs autres substances utilisées pour le traitement de la migraine.

10. Utilisation selon la revendication 9, dans laquelle l'autre substance est le sumatriptan ou un autre composé du groupe des triptans, l'ergotamine ou un dérivé de celle-ci, un bêtabloquant, un anti-épileptique, un analgésique, un anti-émétique ou un antagoniste du calcium.

11. Utilisation selon la revendication 1, dans laquelle le principe actif est administré sous la forme d'une combinaison libre ou fixe avec des vitamines, des antioxydants et/ou des cofacteurs biologiquement fonctionnels.
